# EUROPEAN PATENT APPLICATION

(11) **EP 1 008 353 A1**
(43) Date of publication of application: **14.06.2000**
(21) Application number: 99917131.7
(22) Date of filing: 23.04.1999
(51) Int. Cl.: A61K 47/04, A61K 47/26, A61K 9/20, A61K 7/00, A23L 1/03, C01B 25/32

(54) **SACCHARIDE-CONTAINING COMPOSITIONS**

(30) Priority: 24.04.1998 JP 11543198; 29.07.1998 JP 21421398
(71) Applicant: FUJI CHEMICAL INDUSTRY CO., LTD., Nakaniikawa-gun, Toyama 930-0397 (JP)
(72) Inventor: YOKOI, Shinichiro Fuji Chemical Industry Co., Ltd., Nakaniikawa-gun Toyama 930-0397 (JP); NISHIMURA, Yoshihiro Fuji Chemical Ind. Co. Ltd., Nakaniikawa-gun, Toyama 930-0397 (JP); TANAKA, Nobukazu Fuji Chemical Industry Co., Ltd., Toyama 930-0397 (JP); IKUSHIMA, Heiji Fuji Chemical Industry Co., Ltd., Nakaniikawa-gun Toyama 930-0397 (JP)
(74) Representative: Albrecht, Thomas, Dr.
(86) International application number: JP9902175
(87) International publication number: WO9955373

(57) **Abstract**

Spray-dried powders containing calcium hydrogen phosphate and saccharides having excellent powder-fluidity and compression-moldability, being quickly disintegrated/dissolved when put into the oral cavity or water in the form of granules, tablets, etc., being useful as fillers, compression-moldability improvers, disintegration aids, administration-facilitating agents, solubilizers in the oral cavity, etc. and being applicable to drugs, foods, etc.

## Description

### TECHNICAL FIELD

The present invention relates to spray-dried powders containing calcium hydrogen phosphate and a saccharide which have a good powder-fluidity and a high compression moldability, which are quickly disintegrated and dissolved when put in the oral cavity or water in the form of granule, tablet, etc., which are useful as excipient, compression moldability-increasing agent, disintegration aid, administration-improver, intraoral quick dissoluble agent, etc., and which can be used in medicines, foods, etc.

### BACKGROUND TECHNOLOGY

Calcium hydrogen phosphate has been used in medicines, foods, etc. as a reinforcing agent for calcium and phosphorus components, an antacid, an excipient for pharmaceutical formulation, for example as an excipient for tablet, a diluting agent for tablet or capsule preparation or as anti-caking agent. Calcium hydrogen phosphate has been utilized as an excipient for medicines, cosmetics, foods, etc. owing to its low hygroscopicity, inertness, little reactivity with medicines and no discoloration being caused by itself.

Calcium hydrogen phosphate has generally been prepared by precipitating it from a slurry of lime and phosphoric acid which has been heated above 80 °C. However, the precipitated calcium hydrogen phosphate is a fine powder having a high density and a very small binding property in itself and hence it could not be applied to direct compaction process of tabletting without incorporating into it a binder such as starch or the like (Japanese Patent Application Laid-Open No. Sho 62-36010 gazette).

That is, the hitherto used calcium hydrogen phosphate taking form of plate crystal particle of 10 µm or more is known to be inferior in binding property and lacking in suitability to be used as an excipient. In order to avoid the problem as stated above, various processes have been proposed for imparting moldability to calcium hydrogen phosphate. For example, there is a process for changing the shape or the crystal form of calcium hydrogen phosphate into other one. As the specific example, calcium hydrogen phosphate is pulverlized into fine granules to give it molding property (Japanese Patent Application Laid-Open No. Sho 62-36010). Also, as disclosed in the present applicant's Japanese Patent Application Laid-Open No. Hei 6-298505 gazette, Japanese Patent Application Laid-Open No. Hei 7-118005 gazette (Patent No. 2700141), U.S. Patent No. 5,486,365 specification (1996), European Patent No. 0 644 156 B1 (1998) and Japanese Patent Application No. Hei 8-293251 (Japanese Patent Application Laid-Open No. Hei 10-120408 gazette), there are some processes wherein a prismatic or scaly crystal formed calcium hydrogen phosphate is spray-dried thereby the crystal form is converted into spherical one to impart the moldability to it. More specifically, a process is wherein the starting material, phosphoric acid is reacted with an alkaline calcium compound or an alkaline metal phosphate is reacted with a calcium compound in the presence of a polyvalent organic acid having a coordination ability to prepare calcium hydrogen phosphate composed of primary particles having a prismatic crystal of 0.1 ∼ 1µm in particle size thereby the moldability is imparted to it.

Another process is wherein the prismatic calcium hydrogen phosphate obtained by the above process or calcium hydrogen phosphate having monoclinic crystalline structure is further subjected to hydrothermal treatment to convert into a scaly calcium hydrogen phosphate having a specific surface area of 20 ∼ 60m²/g, a static apparent specific volume of 5 ml/g or more, a primary particle diameter of 0.1 ∼ 5µm and an average agglomerated secondary particle diameter of 2 ∼ 10 µm or wherein said scaly calcium hydrogen phosphate is spray-dried for convertion into spherical one for imparting the moldability to it. Or further another process is wherein the scaly calcium hydrogen phosphate and a cellulose derivative such as sodium carboxymethylcellulose, methylcellulose, hydroxyethyl cellulose or the like as a binder are incorporated into a suspension to be spray-dried for preparing a spherical material having more functional property.

Also, a process was reported for imparting the moldability to calcium hydrogen phosphate by adding binders such as starch, polyacrylic acid and the like to it.

However, these calcium hydrogen phosphates hitherto used cannot yet be said to have a completely satisfactory charateristic properties as excipient, and therefore there has been required further improvement to be achieved in a easy handling including molding property and physical property of powders.

### DISCLOSURE OF THE INVENTION

An object of the present invention is to provide a powdery composition containing calcium hydrogen phosphate and a saccharide which have a good powder-fluidity and a high compression moldability, which are quickly disintegrated and dissolved when put in the oral cavity or water in the form of granule, tablet, etc., which are useful as excipient, compression moldability-increasing agent, disintegration aid, administration-improving agent, intraoral quick dissoluble agent, etc., and which can be used in medicines, foods, etc.

As a result of having ardently studied in order to solve the foregoing object, the present inventors have found that a powdery composition which may be obtained by spray-drying a suspension comprising water medium, calcium hydrogen phosphate and a saccharide has greater moldability, taking feeling and water inducting ability or water adsorbability (calcium hydrogen phosphate itself which is present in the composition has a micro-porous structure for water inducting or water adsorbing structure and further the composition obtained through spray-drying has numerous and fine cavities which become water conducting structure) in comparison with spray-dried preparation of calcium hydrogen phosphate alone. The present invention was completed based on this finding.

That is, the invention involving claim 1 is a powdery composition containing a saccharide and calcium hydrogen phosphate which has been prepared by spray-drying a suspension comprising water medium, calcium hydrogen phosphate and a saccharide, the invention involving claim 2 is a composition containing a saccharide and calcium hydrogen phosphate wherein calcium hydrogen phosphate present in the suspension to be spray-dried is a scaly calcium hydrogen phosphate, the invention involving claim 3 is a composition containing a saccharide and calcium hydrogen phosphate wherein the saccharide is erythritol, the invention involving claim 4 is a process for preparing a spherical and powdered composition containing a saccharide and calcium hydrogen phosphate which is characterized by spray-drying an aqueous suspension comprising calcium hydrogen phosphate and a saccharide, the invention involving claim 5 is an excipient comprising as the main ingredient the composition containing a saccharide and calcium hydrogen phosphate as claimed in claim 1, 2 or 3, the invention involving claim 6 is a compression moldability-improving agent for a pharmaceutical formulation which comprises as the main ingredient the composition containing a saccharide and calcium hydrogen phosphate as claimed in claim 1, 2 or 3, the invention involving claim 7 is a disintegration aid comprising as the main ingredient the composition containing a saccharide and calcium hydrogen phosphate as claimed in claim 1, 2 or 3, the invention involving claim 8 is an administration-improving agent comprising as the main ingredient the composition containing a saccharide and calcium hydrogen phosphate as claimed in claim 1, 2 or 3, the invention involving claim 9 is an intraoral instantaneous (quick) dissoluble agent comprising as the main ingredient the composition containing a saccharide and calcium hydrogen phosphate as claimed in claim 1, 2 or 3, and the invention involving claim 10 is an intraoral instantaneous (quick) dissoluble agent which is characterized by containing further a saccharide in addition to the composition containing a saccharide and calcium hydrogen phosphate as claimed in claim 1, 2 or 3.

The followings illustrate a powdery composition containing calcium hydrogen phosphate and a saccharide which may be prepared by spray-drying a suspension comprising water medium, calcium hydrogen phosphate and a saccharide according to the present invention (hereinafter, referred to as "the composition") in detail.

The composition has superior moldability to either the spray-dried product resulted from an aqueous suspension of calcium hydrogen phosphate alone or one resulted from an aqueous suspension of saccharide alone. For example, when compressed into a tablet form, there may be obtained a tablet having higher hardness than the tablet made from the spray-dried product of calcium hydrogen phosphate alone or saccharide alone. Even when it is mixed with other excipient, the lowering in hardness is little. Also, the spray-dried product comprising calcium hydrogen phosphate alone has particular rough feeling to the tongue when being taked. Contrary thereto, the composition of the present invention has no rough feeling and has refreshing sweetness, thus taking feeling is remarkably improved. In general, the use of a sugar alcohol gives refreshing sensation when it is put in the oral cavity. Among sugar alcohols, especially erythritol and xylitol are preferred.

On the other hand, when an aqueous solution of saccharide is spray-dried or when it is spray-dried in the state of a medicine being dispersed or dissolved therein is spray-dried, the primary particles of saccharide formed by spray-drying melt partially at the heat temperature during spray-drying and subsequently the melted saccharide adheres to the inner wall of a spray dryer or an aggregation occurs through the molten state of saccharide. For this reason, not only the yield (recovery yield) of the spray-dried material is reduced but also the fluidity is not said to be good owing to unevenness in the particle size.

By preparing and spray-drying an aqueous suspension of a saccharide in the presence of calcium hydrogen phosphate or an aqueous suspension of a saccharide, a medicine and/or other antacids in the presence of calcium hydrogen phosphate, there may be achieved advantages that not only saccharide does not melt, but also there may be obtained in a high yield composition containing saccharide having the desired particle size and a good fluidity. Indeed, as a means to prevent a saccharide from being molten during spray-drying, for example to lower the inlet and outlet temperatures as the spay-drying condition is considered. In this case, it can not be said to be efficient because the treatment time becomes long and this impairs the feature of the spray-drying process that the drying may be conducted instantaneously and continuously in a large amount.

The composition involving in the present invention is an amorphous white powdery material which may be obtained by suspending calcium hydrogen phosphate in an aqueous solution of a saccharide e.g. erythritol and then spray-drying the suspension, for example under the condition of an inlet temperature of about 250 °C and an outlet temperature of 60 ∼ 120 °C, preferably 70 ∼ 90 °C for example with a nozzle or a disk spray-dryer according to the conventional manner.

The particle size of the composition involving in the present invention is not particularly limited. There may be obtained one having the desired particle size by varying the preparing condition such as the particle size, the amount and the suspension concentration of calcium hydrogen phosphate to be used or the kind, the amount and the solution concentration of saccharide to be used. The calcium hydrogen phosphate to be used in the present invention may be either dry or wet powdery one which may be obtained by filtering the calcium hydrogen phosphate formed by the reaction (hereinafter, referred to as "undried product"). The dry product may be obtained by spray-drying the undried product having a primary particle size of 0.1 ∼ about 1µ described in Japanese Patent No. 2700141 or Japanese Patent Application No. Hei 8-293251 (Japanese Patent Application Laid-Open No. Hei 10-120408 gazette). Also, as to the undried product its suspension may be used as it is, and mixed with an aqueous solution or a suspension comprising a saccharide, and the mixture may be subjected to spray-drying, thereby obtaining spherical particles.

That the composition has an excellent moldability may be evidenced e.g. by a corn starch load test etc. according to the conventional manner. For example, when the hitherto used calcium hydrogen phosphate is compounded with 20 % of corn starch and compressed into a tablet form under a pressure of 3000 kg, the hardness of the resultant tablet decreases by about 50 % as compared with that of control tablet where no corn starch is added. Contrary thereto, the composition of the present invention is still significantly excellent in moldability even when combined with 30 % of corn starch. Also, in case where the composition of the present invention has been used for tabletting, the residual wall surface pressure and maximum release force are smaller than those of the spray-dried product of calcium hydrogen phosphate alone, and its action toward a punch and a die of tabletting machine becomes small so that the improvement in the tabletting suitability can be realized (see Examples described later).

The composition of the present invention enables the moldability of calcium hydrogen phosphate and the capacity of loading or mixture of drugs to increase, and it enables the wall surface friction to decrease.

The combining ratio of calcium hydrogen phosphate to saccharide such as erythritol in the composition of the present invention is not particularly limited. However, 0.5 ∼ 99.5 % by weight of calcium hydrogen phosphate to 99.5 ∼ 0.5 % by weight of erythritol as an example of saccharide, more preferably, 40 ∼ 99.5 % by weight of calcium hydrogen phosphate to 60 ∼ 0.5 % by weight of erythritol.

Even by increasing the compression tabletting pressure, any effect by varing of the foregoing combining ratio on the residual wall surface pressure can not be seen. This suggests avoidance of the influence of the foregoing combining ratio on the tablet troubles such as sticking etc.

In direct compression tabletting of the composition alone of the present invention, when compression tabletting pressure of 500 kgf is applied there may be obtained a tablet having a hardness of 5 ∼ 7 kg. Also, although the increase in the tablet hardness by application of high compression tabletting pressure is observed, any prolongation in the disintegration time is not recognized.

The results such as apparent density/tap density, water content, particle size distribution, stability, etc. which may be obtained by using the composition of the present invention lie within the normal range for the excipient to be generally conducted in a direct tabletting procedure and rather indicate a good compression moldability, fluidity, taking feeling and whiteness.

Also, since the composition of the present invention has a little friction resistance there can be avoided the troubles derived from the abrasion of a punch and a die in the tablet machine which is usually used in the tabletting.

The followings illustrate a process for preparing the composition of the present invention.

The composition of the present invention may be obtained as an amorphous white powder by suspending calcium hydrogen phosphate in an aqueous solution of a saccharide and then spray-drying the suspension for example under the condition of an inlet temperature of 200 °C and an outlet temperature of 100 °C for example with a nozzle or a disk spray-dryer according to the conventional manner.

As to the particle size of the composition, it may be adjusted as desired by properly selecting the suspension concentration, spray operation, drying condition, etc, as stated previously.

As calcium hydrogen phosphate which may be used in the present invention, for example calcium hydrogen phosphate "ATB" (a trade name, a product of Rhone-Poulenc Co., Ltd., "DiTAB" (a trade name, a product of Nikken Chemical Co., Ltd.), "GS Calica" (a trade name, a product of Kyowa Chemical Co., Ltd.), a spherical calcium hydrogen phosphate "Fujicalin SG" (a trade name, a product of Fuji Chemical Industry Co., Ltd.), a scaly calcium hydrogen phosphate "Fujicalin" (a trade name, a product of Fuji Chemical Industry Co., Ltd.), or the calcium hydrogen phosphate and anhydrous calcium hydrogen phosphate obtained by the known process may be employed. Anhydrous calcium hydrogen phosphate may be either calcium dihydrogen phosphate or calcium secondary phosphate. Calcium hydrogen phosphate may be used in combination with anhydrous calcium hydrogen phosphate. A spherical calcium hydrogen phosphate "Fujicalin SG" and a scaly calcium hydrogen phosphate "Fujicalin" are preferred.

Also, the spherical calcium hydrogen phosphate which may be obtained by spray-drying the foregoing scaly calcium hydrogen phosphate is hardly soluble in water and neutral, and does not react with the main ingredient, and therefore it is not adversely affected on the main ingredient and the formulation. And, it is easy to handle owing to its good fluidity and miscibility. Furthermore, it has a good affinity to water so that it is easily suspensible in water and a slurry preparation may be conducted conveniently and simply.

As the saccharide, there are monosaccharide, oligosaccharide and polysaccharide. For example, sugar alcohols such as erythritol, mannitol, sorbitol, xylitol and the like, maltitol, maltitose, lactitol, spray-dried lactose, granular lactose, anhydrous lactose, lactose, refined sucrose, reducing maltose, thick malt syrup, cane sugar (sucrose), fruit sugar (fructose), arabitol, erythrose, glucose, fructose, xylose, arabinose and the like may be taken.

The composition of the present invention may be used, when being spray-dried, in combination with inorganic or organic solid carriers for formulation which has been conventionally used in the preparation of medicines, for example starches or celluloses such as crystalline cellulose, hydroxypropyl methylcellulose, hydroxypropyl cellulose, carboxymethylcellulose, hydroxypropylstarch, sodium carboxymethylstarch, sodium croscarmellose, calcium carboxymethylcellulose and the like.

The selection of the kind and amount of saccharide to be added may be adequately made depending on the objects of the main ingredient and the formulation, for example the improvement in molding property and disintegration, intraoral disintegratable formulation suited to therapeutic region for the aged person, and the like. Also, the particle size of saccharide may be adjusted as desired. Sugar alcohols such as erythritol, sucrose, fruit sugar (fructose), xylitol, mannitol, arabitol and the like are easily soluble in water and hot ethanol, and they may be used as the solution or the suspension. The amount used of sugar alcohols is not particularly limited.

In case of non-calory saccharides such as erythritol and xylitol, it is especially desirable to use them in the anti-diabetic formulation.

Also, erythritol, xylitol, etc. may be preferably used in the intraoral instantaneous (quick) dissoluble formulation owing to their taste with refreshing sensation (cooling sensation).

The composition of the present invention comprising saccharaide and calcium hydrogen phosphate can be molded into tablets by the known processes. The tablts may be obtained by direct compression tabletting the composition alone or in combination with one or more of pharmaceutically active ingredients, in addition with binders, disintegrants, colorants, flavoring agents, diluents, lubricants etc. The tablets may be molded into any forms as desired including circular, elliptic and caplet-like forms irrespective of the size and shape.

The composition of the present invention may also be used as a filler in powderes, fine granules, granules, capsules, etc., or as an absorbent for oily materials such as fragrances.

The composition of the present invention may be molded in the forms which are suitable for oral or non-oral administration in combination with inorganic or organic solid carriers for formulation which have been conventionally used in the preparation of medicines, for example excipients such as starch, crystalline cellulose, hydroxypropyl methylcellulose, calcium carboxymethylcellulose, hydrogenated oil, talc and the like; binders such as acacia, hydroxypropyl cellulose, alginic acid, gelatin. polyvinyl-pyrrolidone, partially alpha starch and the like; lubricants such as stearic acid, magnesium stearate, calcium stearate, talc, hydrogenated vegetable oil and the like; disintegrants such as modified atarch, calcium carboxymethylcellulose, hydroxypropyl methylcellulose, hydroxypropyl cellulose, sodium carboxymethylstarch and the like; nonionic surfactant, etc.

The forms which are suitable for oral administration include tablets, capsules, powders, fine granules, granules or their sugar coating, film coatings and the like. Ones which are suitable for local application include an ointment, a suppository, a plaster or a patching agent, absorbent for cream and viscosity-increasing agent, a gel, etc.

The intraoral instantaneous (quick) dissolvable agent of the present invention is an intraoral quick dissoluble formulation which contains the composition of the present invention as a functional excipient and which may be prepared by the general direct compression tabletting method.

For preparing the tablets, the composition of the present invention may be used in combination with saccharides which have been usually used.

This formulation may be obtained from the composition of the present invention or one wherein an adequate saccharide and other additives have been further incorporated by direct compression tableting with a tabletting tester.

In case where the tablets are prepared using the composition of the present invention, the combining ratio is not particularly limited. For the moldability, however, it is desirable to incorporate 5 % by weight ∼ 99 % by weight, preferably 10 % by weight ∼ 70 % by weight of the composition per tablet weight. For giving a refreshing sensation to the oral cavity, it is preferable to incorporate 40 % by weight ∼ 99 % by weight of the composition per tablet weight. For preparation of intraoral instantaneous (quick) dissoluble agent, it is preferable to incorporate 50 % by weight ∼ 95 % by weight of the composition per tablet weight.

The obtained tablets are instantaneous (quick) dissoluble. As a result of disintegration test conducted under condition not to chew them in the oral cavity, there may be prepared ones which are disintegrated within 1 minute. In addition, by combining the composition of the present invention with an adequate saccharide, there can be conveniently and simply obtained tablets having a disintegration time of 1 minute or less in all cases. The compression tabletting pressure and the tablet hardness are varied depending on the kind and amount of the composition to be used and not particularly limited. For example, by application of a pressure ranged 100∼ 1000 kg there can be obtained a tablet having a hardness of 1.1 ∼ 14.5 kg. In case where disintegrants which have been generally used are added to the composition of the present invention, the disintegration time may be further shortened (see Exambles described later).

In case where the composition which may obtained in the present invention is used as an excipient, etc. for pharmaceutical preparation, the examples of its pharmaceutically active ingredient include peripheral nervous system agent, antipyretic, analgesic anti-inflammatory agent, central nervous system drugs such as hypnic sedative, psychic and nervous system agent, etc.; peripheral nervous system agents such as skeletal muscle relaxants, autonomic nervous system agents, etc.; cardiovascular and circulatory system agents such as cardiotonics, anti-arrhythmic drugs, diuretics, vasodilators; respiratiry system agents such as bronchodilators, antitussives, etc.; gastrointestinal system agents such as digestive, intestinal function-controlling agent, antacids, etc.; metabolic drugs such as hormones, antihistaminic agents, vitamins, etc.; anti-ulcer agents, antibiotics, chemotherapeutic agents, crude drug-extracts and the like.

The composition of the present invention is suitable for use as an excipient in (1) medicines for the aged person, for example cardiovascular and circulatory system such as hypotensive drugs; antilipidemics and the like, (2) medicines having poor moldability, or (3) medicines having bad taste such as bitter taste.

The kind of a pharmaceutically active ingredient is not particularly limited in the present invention. As specific examples of the pharmaceutically active ingredient, there may be taken an active agent for cold remedy, an active agent for rhinitis and the like. Examples of the active agent for cold remedy include antipyretic, analgesic, anti-inflammatory agent, vasodilators, antihistaminic agents, antitussives, expectorants, antitussive expectorants, vitamins, Chinese medecine extracts and the like. Examples of an active agent for the active agent for rhinitis include sympathomimetics, sympatholytics, anti-allergenic agents, anti-inflammatory agents and the like. Examples of antipyretic, analgesic anti-inflammatory agents include aniline derivatives such as acetaminophen, phenacetin, lefetamine hydrochloride and the like; salicylic acid derivatives such as ethenzamide, sasapyrine, methyl salicylate, phenyl salicylate, sodium salicylate, choline salicylate, aspirin, aspirin aluminum and the like; pyrazolone derivatives such as isopropylantipyrine, sulpyrin, phenylbutazone, ketophenylbutazone, antipyrine, aminopyridine and the like; propionic acid derivatives such as ibuprofen, ketoprofen, oxaprozin, naproxen, fenoprofen calcium, tiaprofenic acid and the like; phenylacetic acid derivatives such as fenbufen, diclofenac sodium, anfenac sodium and the like; indoleacetic acid derivatives such as indomethacin, indomethacin farnesil, proglumetacin maleate, sodium tolmetin and the like; anthranilinoacetic acid derivatives such as mefenamic acid, flufenamic acid. tolfenamic acid and the like; oxicam derivatives such as piroxicam, ampiroxicam, tenoxicam and the like; benzydamine hydrochloride, epirizole (mepirizole), tinoridine hydrochloride, tiaramide hydrochloride, etc., anti-inflammatory enzyme preparations, serrapeptase, lysoxyme chloride and the like. These antipyretic, analgesic anti-infammatory agents may be used singly or in combination of two or more.

Examples of bronchodilators include ephedrine hydrochloride, dl-methylephedrine hydrochloride, dl-methylephedrine hydrochloride saccharinate, isoprenaline hydrochloride, isoproterenol sulfate, methoxyphenamine hydrochloride, orciprenaline sulfate, chlorprenaline hydrochloride, trimetoquinol hydrochloride, salbutamol sulfate, terbutaline sulfate, hexoprenaline sulfate, formoterol fumarate, fenoterol hydrobromide, procaterol hydrochloride, pirburol hydrochloride, clenbuterol hydrochloride, mabuterol hydrochloride, xanthine derivatives such as aminophylline, theophylline, diprophylline, proxyphylline and the like; anti-cholinergics such as flutropium bromide, oxitropium bromide and the like. Examples of antihistaminic agents include ethanolamine antihystaminic agents such as diphenhydramine and the like; propylamine antihystaminic agents such as dl-chloropheniramine maleate, d-chloropheniramine maleate and the like; phenothiazine antihystaminic agents such as alimemazine tartrate, isothipendyl hydrochloride, promethazine hydrochloride, mequitazine, and the like; diphenylpyraline, carbinoxamine maleate, clemastine fumarate, iproheptine hydrochloride, homochlorcyclizine hydrochloride, cycloheptazine hydrochloride, dimetindene maleate, triprolidine hydrochloride, etc.

Examples of antitussives include codeines such as codeine phosphate, dihydrocodeine phosphate and the like; dextromethorphan hydrobromide, cloperastine, noscapine dimemorfan, oxeladin, pentoxiverin citrate, eprazinone hydrochloride, clobutinol hydrochloride, isoaminile citrate, fominoben hydrochloride, clofedanol hydrochloride, benproperine phosphate, hydrocotarnine hydrochloride, sodium dibunate, etc.

Examples of expectorants include potassium guaiacolsulfonate; cysteine derivatives such as carbocysteine, L-ethylcysteine hydrochloride, L-methylcysteine hydrochloride, acetylcysteine and the like; bromhexine, ambroxol hydrochloride, etc. Examples of antitussive expectorants include guaiphenesin, tipepidine, oxymethebanol, alloclamide hydrochloride, carbetapentane citrate, trimetoquinol hydrochloride, methoxyphenamine and the like. Some of the pharmaceutically active ingredients exemplified as antitussives, expectorants and antitussive expectorants described above show antititussive and/or expectorant actions. Examples of vitamins include vitamin B₁, its derivatives or salt thereof such as fursultiamine, fursultiamine hydrochloride, prosultiamine, octotiamine, tiaminedisulfide, bisbentiamine, bisbutythiamin, bisibutiamine, benfotiamine, cetotiamine hydrochloride and the like; vitamin B₂, its derivatives or salts thereof such as riboflavin, sodium riboflavin phosphate, sodium flavin-adenine-dinucleotide, riboflavin lactate and the like; vitamin C, etc.

The drugs in the present invention may also include pharmacologically acceptable salts of the forgoing drugs with an inorganic or an organic acid. Examples of an acid include hydrochloric acid, phosphoric acid, sulfuric acid, etc., tannic acid, citric acid, fendizoic acid, hibenzic acid, citric acid, etc.

Examples of Chinese medicine extract include licorice, cinnamon, puerariae radix, apricot kernel, bupleuri radix, ginger, ginseng, dried orange peel, pinelliae tuber and the like.

Examples of sympathomimetic agents (α-receptor stimulants) which constitute drugs for rhinitis include decongestands such as phenylpropanolamine hydrochloride, pseudoephedrine hydrochloride, phenylephrine hydrochloride and the like. Examples of parasympatholytics include belladonna alkaloids and the like. Examples of anti-allergic and anti-inflammatory drugs include tranexamic acid and the like. These active ingredients may be used singly or in combination of two or more.

If necessary, the active ingredients described above may be used in association with caffeines such as anhydrous caffeine, caffeine sodium benzoate, caffeine citrate, caffeine (monohydrate) and the like; minerals and amino acids thereof such as magnesium hydroxide, magnesium oxide, magnesium carbonate, magnesium silicate, aluminium sulfate, synthetic hydrotalcite [Alkamac (a trade name)], synthetic aluminium silicate, dihydroaluminium aminoacetic acid salt, aluminium hydroxide gel, magnesium alumino metasilicate, magnesium alumino silicate, aluminium hydroxide-calcium hydrogen phosphate co-precipitate, sucralfate and the like.

The features of the composition obtained by the present invention are shown as follows: there can be taken (1) improvement in the moldability of a tablet, (2) improvement of fluidity in tabletting (specifaclly, direct compression molded tablet has a good homogeneity with respect to the ingredient content), (3) improvement in taking feeling (refreshing sensation and/or cooling sensation), (4) improvement in disintegration property and (5) usefulness as intraoral instantaneous (quick) dissoluble tablet.

### BRIEF EXPLANATION OF THE DRAWINGS

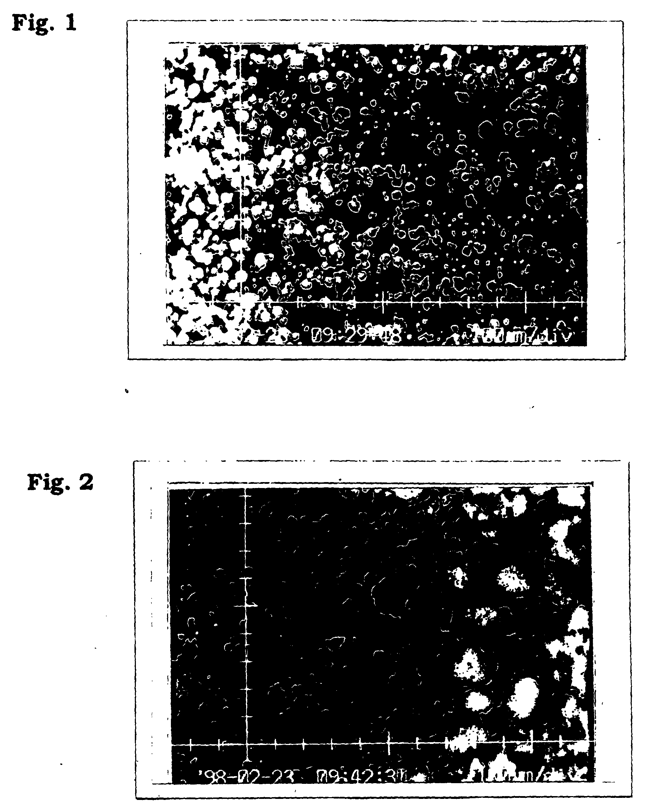

Fig. 1 is a scanning electron microgragh (SEM) of the spray-dried product comprising calcium hydrogen phosphate/erythritol (75/25 % by weight) obtained in Example 3.
Fig. 2 is a SEM of the spray-dried product comprising calcium hydrogen phosphate/erythritol (75/25 % by weight) obtained in Example 6.

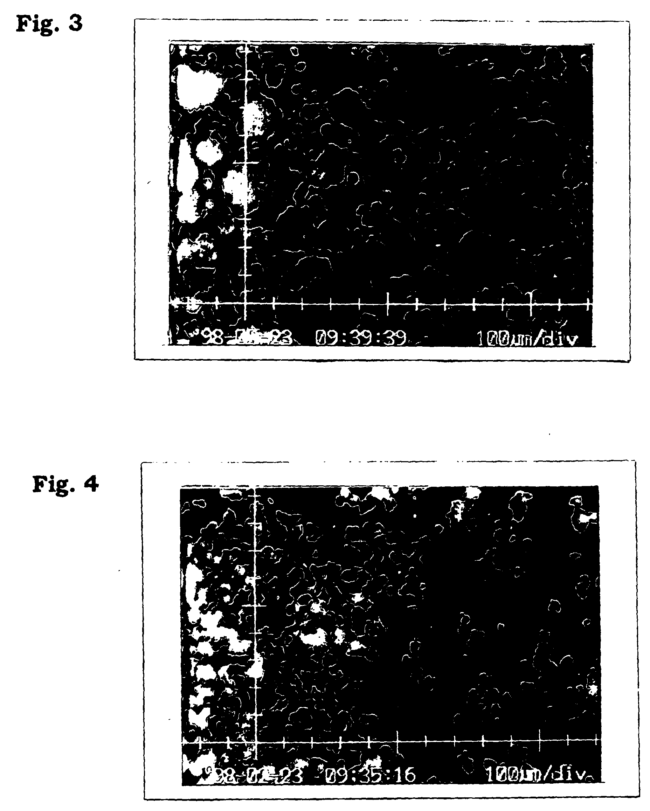

Fig. 3 is a SEM of the spray-dried product comprising calcium hydrogen phosphate/erythritol (75/25 % by weight) obtained in Example 7.
Fig. 4 is a SEM of the spray-dried product comprising calcium hydrogen phosphate/erythritol (75/25 % by weight) obtained in Example 8.

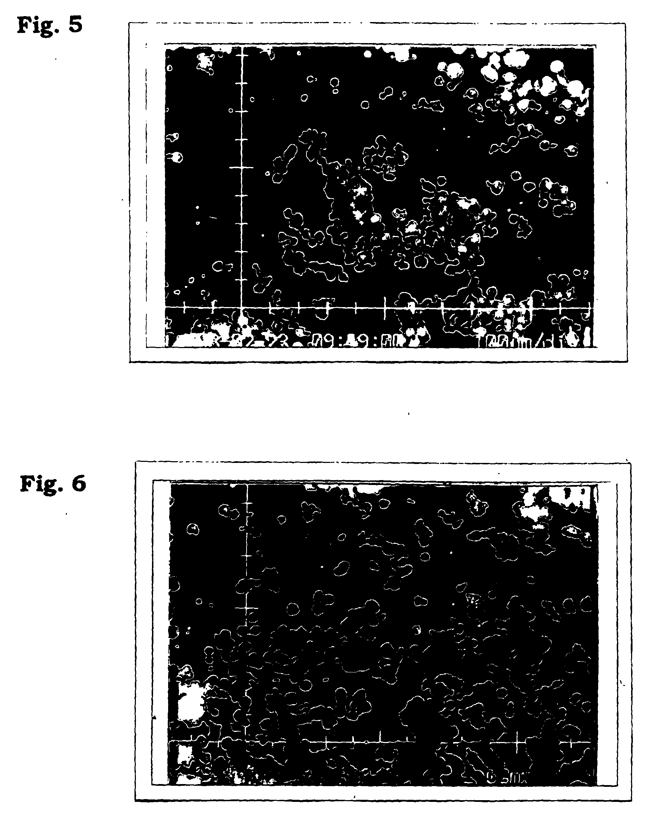

Fig. 5 is a SEM of the spray-dried product comprising calcium hydrogen phosphate/lactose (75/25 % by weight) obtained in Example 9.
Fig. 6 is a SEM of the spray-dried product comprising calcium hydrogen phosphate/lactose (50/50 % by weight) obtained in Example 10.

### THE BEST MODE FOR CARRYING OUT THE INVENTION

The followings illustrate uses of the composition obtained in the present invention in detail.

### Example 1 : Preparation of a composition wherein the combining ratio of calcium hydrogen phosphate to erythritol is 95 : 5

10 Grams of erythritol was dissolved in 1 liter of water and to the aqueous solution under stirring was added 190 g of spherical calcium hydrogen phosphate ("Fujicalin SG") was added. The resultant suspension was spray-dried using a centrifuge-type atomizer at an inlet temperature of 250 °C and an outlet temperature of 100 ∼ 110°C to obtain 180 g of white spherical granular material.

### Examples 2 ∼ 5

There were prepared a series of compositions wherein the combining ratio of spherical calcium hydrogen phosphate to erythritol is 85 : 15 ∼ 50 : 50 in a similar manner as in Example 1.

The results are shown in table 1.

**Table 1**

| Example No. | Amount mixed of Calcium Hydrogen Phosphate (% by weight) | Amount mixed of Erythritol (% by weight) |
|---|---|---|
| 2 | 85 | 15 |
| 3 | 75 | 25 |
| 4 | 65 | 35 |
| 5 | 50 | 50 |

### Physico-chemical test:

Tablets for "Corn Starch Load Test" were prepared from the compositions obtained in Examples 1 ∼ 5 for evaluation of the moldability of said compositions and the comparison test was conducted for the physical property such as the tablet hardness. The "Corn Starch Load Test" is a method to evaluate such moldability by adding the composition of the present invention or calcium hydrogen phosphate to a corn starch which has no molding property in itself as how to extent that the resultant mixture can be molded into a tablet form.

### Example 6

Calcium hydrogen phosphate "ATAB" (a trade name, a product of Rhone-Poulenc Co., Ltd. and erythritol were used to prepare a specimen in a similar manner as in Example 1, and then tablets for corn starch load test were prepared similarly as in the above.

### Example 7

Calcium hydrogen phosphate "DiTAB" (a trade name, a product of Nikken Chemical Co., Ltd.) and erythritol were used to prepare a specimen in a similar manner as in Example 1, and then tablets for corn starch load test were prepared similarly as in the above.

### Example 8

Calcium hydrogen phosphate "GS Calice" (a trade name, a product of Kyowa Chemical Co., Ltd.) and erythritol were used to prepare a specimen in a similar manner as in Example 1, and then tablets for corn starch load test were prepared similarly as in the above.

### Preparation of a tablet

8 Grams of the total amount weighed with respect to a mixture of the composition obtained in Example 3, a corn starch (a product of Nippon Corn Starch Co., Ltd.) and magnesium stearate (a product of Nippon Oils & Fats Co., Ltd.)in the combining ratio (% by weight) shown in the following table 2.

The composition obtained in Example 3 was used for Experimental Examples 1 ∼ 6. A scaly calcium hydrogen phosphate "Fujicalin" which may be prepared by the process disclosed in Japanese Patent Application Laid-Open No. Hei 7-118005 as the hitherto used product was used for Experimental Examples 7 ∼ 12. And the compositions obtained in Examples 6 ∼ 8 were used for Experimental Examples 13 ∼ 15, respetively.

**Table 2**

| Exp. No. | Composition | Content (% by wt.) | Content of Corn Starch (% by wt.) | Magnesium Stearate (% by wt.) |
|---|---|---|---|---|
| 1 | Example 3 | 99 | 0 | 1 |
| 2 | Example 3 | 89 | 10 | 1 |
| 3 | Example 3 | 79 | 20 | 1 |
| 4 | Example 3 | 69 | 30 | 1 |
| 5 | Example 3 | 59 | 40 | 1 |
| 6 | Example 3 | 49 | 50 | 1 |
| 7 | Hitherto Used Product | 99 | 0 | 1 |
| 8 | Hitherto Used Product | 89 | 10 | 1 |
| 9 | Hitherto Used Product | 79 | 20 | 1 |
| 10 | Hitherto Used Product | 69 | 30 | 1 |
| 11 | Hitherto Used Product | 59 | 40 | 1 |
| 12 | Hitherto Used Product | 49 | 50 | 1 |
| 13 | Example 6 | 99 | - | 1 |
| 14 | Example 7 | 99 | - | 1 |
| 15 | Example 8 | 99 | - | 1 |
| Note) As the hitherto used product in Experimental Examples 7 ∼ 12, a scaly calcium hydrogen phosphate alone was used. | | | | |

Each of the components in the combining ratio shown in table 2 was placed in a dried polyethylene bag and the bag was sealed not so as to absorb moisure. After mixing sufficiently, the mixture was compressed into a tablet form using "Tabletting Tester-SK-02" (a trade name, a product of Sankyo Biotech Co., Ltd.) to estimate the relationship between the compression tableting pressure and the tablet hardness.

The tablets each weighing 600 mg and having a diameter of 11.3 mm were prepared at a tabletting velocity of 10 mm/min under compression tableting pressures of 500 kgf, 1000 kgf, 2000 kgf and 3000 kgf.

The results are shown in Table 3.

**Table 3**

| Exp. No. | Compression Tabletting Pressure | | | |
|---|---|---|---|---|
| | 500 kgf | 1000 kgf | 2000 kgf | 3000 kgf |
| 1 | 12.3 | 28.3 | 46.6 | 71.9 |
| 2 | 7.9 | 20.1 | 41.0 | 54.6 |
| 3 | 6.9 | 19.7 | 39.9 | 57.3 |
| 4 | 4.8 | 14.7 | 35.7 | 49.4 |
| 5 | 2.9 | 11.6 | 24.9 | 34.3 |
| 6 | 1.3 | 5.7 | 13.2 | 19.6 |
| 7 | 11.8 | 22.8 | 35.7 | 43.9 |
| 8 | 8.4 | 15.9 | 30.4 | 35.8 |
| 9 | 4.9 | 9.3 | 21.5 | 23.8 |
| 10 | 2.5 | 5.2 | 11.8 | 15.3 |
| 11 | 2.0 | 5.5 | 10.1 | 12.2 |
| 12 | 1.0 | 3.4 | 8.1 | 7.5 |

It can be seen from the results shown in table 3 that the tablets made from the composition of the present invention have higher hardness even under all compression tabletting pressures applied and significantly increased hardness especially under the compression tabletting pressure region of 1000 Kg or more in comparison with the control tablets made from the hitherto used product alone-formulation wherein the corn starch content is the same as that in the composition of the present invention.

① The residual wall surface stress (Pwr), ② the release force and ③ the maximum release force of the above samples were measured using a "Tabletting Tester-SK-02" apparatus.

**Table 4**

| Residual Wall Surface Stress [Pwr: unit (kg/cm²) | | | | |
|---|---|---|---|---|
| Exp. No. | Compression Tabletting Pressure | | | |
| | 500 kgf | 1000 kgf | 2000 kgf | 3000 kgf |
| 1 | 107 | 188 | 318 | 449 |
| 2 | 94 | 150 | 229 | 292 |
| 3 | 92 | 121 | 153 | 162 |
| 4 | 76 | 99 | 101 | 100 |
| 5 | 63 | 79 | 73 | 60 |
| 6 | 68 | 69 | 60 | 56 |
| 7 | 144 | 231 | 405 | 557 |
| 8 | 122 | 197 | 321 | 427 |
| 9 | 106 | 164 | 250 | 302 |
| 10 | 93 | 144 | 193 | 225 |
| 11 | 89 | 128 | 174 | 182 |
| 12 | 94 | 110 | 134 | 143 |
| 13 | 63 | 121 | 274 | 375 |
| 14 | 59 | 101 | 194 | 284 |

As is apparent from the results shown in Table 4, the composition of the present invention gives tablets having less wall surface abrasion in all of the formulations and compression tabletting pressures applied, then better compression moldability as compared with the control calcium hydrogen phosphate alone wherein the corn starch content is the same as that in the composition of the present invention.

**Table 5**

| Maximum Release Force (Pej) | | | | |
|---|---|---|---|---|
| Exp. No. | Compression Tabletting Pressure | | | |
| | 500 kgf | 1000 kgf | 2000 kgf | 3000 kgf |
| 1 | 10.9 | 19.8 | 37.5 | 54.9 |
| 2 | 9.8 | 15.6 | 24.7 | 32.9 |
| 3 | 7.6 | 11.0 | 15.7 | 18.3 |
| 4 | 6.4 | 10.1 | 10.6 | 11 0 |
| 5 | 5.9 | 7.6 | 7.7 | 7.5 |
| 6 | 7.3 | 6.9 | 6.2 | 5.6 |
| 7 | 30.3 | 43.7 | 74.5 | 92.6 |
| 8 | 17.3 | 27.2 | 44.9 | 61.0 |
| 9 | 13.9 | 20.6 | 31.2 | 37.0 |
| 10 | 11.2 | 17.4 | 22.4 | 25.7 |
| 11 | 10.0 | 14.2 | 17.1 | 17.4 |
| 12 | 8.9 | 11.7 | 12.6 | 11.4 |
| 13 | 5.6 | 13.0 | 29.5 | 48.1 |
| 14 | 7.0 | 14.3 | 28.9 | 41.8 |
| 15 | 5.6 | 11.5 | 24.3 | 39.5 |

The composition of the present invention gives tablets having less realease force as compared with the calcium hydrogen phosphate alone-formulation.

### Experimental Examples 16 ∼ 19

As a sample, 10 g of each recipe weighed which comprises 97 % by weight of the spray-dried product wherein the combining ratio of a spherical calcium hydrogen phosphate "Fujicalin SG" to erythritol is 95 : 5. (Example 1), 85 : 15 (Example 2), 75 : 25 (Example 3) or 65 : 35 (Example 4), 2 % by weight of sodium croscarmellose and 1 % by weight of magnesium stearate (a product of Nippon Oils & Fats Co., Ltd.) , and was mixed in a dried polyethylene bag to prepare a specimen for a tabletting tester, which was molded into tablets for Experimental Examples 16 ∼ 19.

**Table 6**

| Exp. No. | Amount of Composition in each of Examples (% by wt.) | Sodium Croscarmellose (% by wt.) | Magnesium Stearate (% by wt.) |
|---|---|---|---|
| 16 | (Example 1) 97 | 2 | 1 |
| 17 | (Example 2) 97 | 2 | 1 |
| 18 | (Example 3) 97 | 2 | 1 |
| 19 | (Example 4) 97 | 2 | 1 |

Test for the relationship between compression tabletting pressure and the tablet hardness was conducted according to a similar manner as in the above.

**Table 7**

| Exp. No. | Compression Tabletting Pressure | | | |
|---|---|---|---|---|
| | 500 kgf | 1000 kgf | 2000 kgf | 3000 kgf |
| 16 | 6.4 | 13.6 | 27.1 | 38.9 |
| 17 | 7.4 | 16.3 | 33.4 | 49.1 |
| 18 | 8.7 | 20.6 | 40.0 | 57.2 |
| 19 | 6.1 | 16.5 | 40.3 | 56.0 |

As can be seen from the results shown in Table 7, in considering the combining ratio of erythritol to calcium hydrogen phosphate application of the compression tabletting pressure of 500 kgf results in the formation of tablets having sufficient hardness required as the plain tablets even in all of the combining ratios, and the tablet hardness increases in propotion to compression tabletting pressure.

The compositions of Experimental Examples 16 ∼ 19 for the residual wall surface stress was tested in a similar manner as previously stated.

As a result, there was recognized a slight effect of the disintegrant on the residual wall surface stress, but there was not recognized any effect of the combining ratio of erythritol to calcium hydrogen phosphate on it.

The compositions of Experimental Examples 16 ∼ 19 for the maximum release force was tested in a similar manner as previously stated.

As a result, there was not recognized any effect of the disintegrant on the release force but there was shown a tendency to diminish effect of the combining ratio of erythritol to calcium hydrogen phosphate on it.

### Disintegration test:

According to the method of the Japanese Pharmacopoeia, three tablets in each test were immersed in the first liquid of the Japanese Pharmacopoeia, and the time required for the form of the tablet to be completely disintegrated was measured.

**Table 8**

| Exp. No. | Compression Tabletting Pressure | | | |
|---|---|---|---|---|
| | 500 kgf | 1000 kgf | 2000 kgf | 3000 kgf |
| 16 | 1.7 | 1.3 | 1.1 | 1.0 |
| | 1.7 | 1.3 | 1.2 | 1.1 |
| | 2.1 | 1.3 | 1.1 | 1.2 |
| 17 | 3.7 | 3.2 | 2.2 | 3.1 |
| | 3.8 | 3.1 | 2.1 | 3.0 |
| | 3.8 | 3.2 | 3.0 | 3.1 |
| 18 | 6.0 | 5.4 | 3.8 | 6.0 |
| | 6.1 | 5.5 | 4.4 | 5.6 |
| | 6.4 | 5.6 | 4.1 | 5.8 |
| 19 | 6.9 | 4.9 | 6.2 | 5.7 |
| | 7.4 | 4.7 | 6.0 | 5.7 |
| | 7.5 | 5.2 | 7.4 | 5.7 |

Disintegration time in the table is shown in minute.

Although there was recognized a tendency to extend the disintegration time with decrease in the amount compounded of calcium hydrogen phosphate or with increase in the amount compounded of erythritol, there was not recognized any effect of the molding pressure on it.

In synthesizing the evaluation results of the moldability and the disintegrability, it can be seen that an improvement in the moldability and adjustment of the disintegrating property may be made by the combining ratio of saccharide to calcium hydrogen phosphate.

### (Evaluation of taking feeling)

A tongue test by six volunteers (three men and three women) in the Company was carried out. Tablets prepared by application of a compression tabletting pressure of 500 kg to the formulation of Experimental Example 19 were used as samples, and their taste, aftertaste, cooling sensation, taking feeling and intraoral dissolution property were evaluated by ranking of "Good" "Ordinal" and "Bad". Tablets consisting of calcium hydrogen phosphate "ATB" alone were used as control.

**Table 9**

| Sample | Taste and Aftertaste (Number of Person) | Cooling Sensation (Number of Person) | Taking Feeling (Number of Person) | Intraoral (Disintegration) Dissolution Property | Total Evaluation |
|---|---|---|---|---|---|
| Tablet Tested | Slight sweet taste and refreshing aftertaste (6 persons) | Preferable owing to cooling sensation (5 persons) | Gradually dissolved in the mouth and not sticky (6 persons) | Dissolved (disintegrated) within 1-2 minutes | Excellent |
| Control Tablet | Tasteless (6 persons) | None (6 persons) | Adhered to teeth and tongue (5 persons) | Dissolved (disintegrated) within 1 minute | Slighyly bad |

Furthermore, the kind and amount of the disintegrant are selected in the combination with the composition of the present invention and followed by forming into a tablet having a sufficient hardness range required as an industrially feasible tablet, thereby instantaneously (quickly) dissoluble tablets suited to the intended use and tablets having the general disintegration degree may be obtained.

### Examples 9 ∼ 10

A spherical calcium hydrogen phosphate "Fujicalin SG" and lactose in the following formulations were spray-dried in a similar manner in Example 1 to prepare the respective calcium hydrogen phosphate-lactose compositions which are designated as samples for Examples 9 ∼ 10.

**Table 10**

| Example No. | Amount of Calcium Hydrogen Phosphate (% by wt.) | Amount of Lactose (% by wt.) |
|---|---|---|
| 9 | 75 | 25 |
| 10 | 50 | 50 |

### Comparative Example 1

Calcium hydrogen phosphate and magnesium stearate were combined so as to become 99 % by weight and 1 % by weight, respectively and the mixture was formed into tablets in a similar manner as stated previously.

### Comparative Example 2

Lactose and magnesium stearate were combined so as to become 99 % by weight and 1 % by weight, respectively and the mixture was formed into tablets in a similar manner as stated previously.

A series of tests were conducted using samples for Examples 9 ∼ 10 and Comparative Examples 1 ∼ 2 to evaluate the relationship between the compression tabletting pressure and any of the tablet hardness, the residual wall surface stress and the maximum release force.

**Table 11**

| Example No. | Compression Tabletting Pressure | | | |
|---|---|---|---|---|
| | 500 kgf | 1000 kgf | 2000 kgf | 3000 kgf |
| Example 9 | 122 | 194 | 328 | 394 |
| Example 10 | 103 | 182 | 297 | 393 |
| Comparative Example 1 | 131 | 215 | 361 | 496 |
| Comparative Example 2 | 95 | 141 | 285 | 343 |

A test was conducted to evaluate the relationship between a compression tabletting pressure and the hardness of the resultant tablets.

From the results shown in the above table, the hardness of the tablets made from the spray-dried lactose-calcium hydrogen phosphate composition is higher than that of tablets made from lactose alone. Thus, it could be confirmed that the composition of the present invention where lactose is concerned increases moldability.

Also, the residual wall surface stress was tested in a similar manner as stated previously.

**Table 12**

| Example No. | Compression Tabletting Pressure | | | |
|---|---|---|---|---|
| | 500 kgf | 1000 kgf | 2000 kgf | 3000 kgf |
| Example 9 | 122 | 194 | 328 | 394 |
| Example 10 | 103 | 182 | 297 | 393 |
| Comparative Example 1 | 131 | 215 | 361 | 496 |
| Comparative Example 2 | 95 | 141 | 285 | 343 |

The calcium hydrogen phosphate-lactose composition decreases in the residual wall surface stress.

**Table 13**

| Maximum release force test | | | | |
|---|---|---|---|---|
| Example No. | Compression Tabletting Pressure | | | |
| | 500 kgf | 1000 kgf | 2000 kgf | 3000 kgf |
| Example 9 | 15 | 26 | 45 | 70 |
| Example 10 | 13 | 33 | 60 | 83 |
| Comparative Example 1 | 41 | 67 | 88 | 93 |
| Comparative Example 2 | 11 | 21 | 43 | 61 |

The maximum release force was tested in a similar manner as stated previously.

With respect to ralease force, the calcium hydrogen phosphate-lactose composition decreases in the residual wall surface stress.

### A process for preparing an intraoral instanteneous (quick) dissoluble (disintegratable) agent

It was prepared by direct compression tabletting of a mixture of the composition of the present invention and saccharide using a tabletting tester.

In the following Examples 11 ∼ 13, there was used the composition obtained in Example 4 wherein a suspension comprising 65 % of calcium hydrogen phosphate and 35 % of erythritol was spray-dried.

### Example 11

A spray-dried powder obtained in Example 4, lactose and magnesium stearate were combined so as to be 60 % by weight, 39 % by weight and 1 % by weight, respectively and mixed together. Thereafter, tablets each weighing 500 mg (tablet diameter:11.3 mm) were obtained. The relationship between molding pressure and hardness, and intraoral disintegration property (without chewing) are shown in the following table.

**Table 14**

| Molding Pressure (kg) | Hardness (kg) | Intraoral Disintegration Time (min) |
|---|---|---|
| 100 | 1.1 | Within 1 minute |
| 200 | 1.2 | Within 1 minute |
| 500 | 2.6 | Within 1 minute |
| 800 | 4.5 | Within 1 minute |

### Example 12

A spray-dried powder obtained in Example 4, erythritol and magnesium stearate were combined so as to be 60 % by weight, 39 % by weight and 1 % by weight, respectively and mixed together. Thereafter, tablets each weighing 500 mg (tablet diameter:11.3 mm) were obtained. The relationship between molding pressure and hardness, and intraoral disintegration property (without chewing) are shown in the following table.

**Table 15**

| Molding Pressure (kg) | Hardness (kg) | Intraoral Disintegration Time (min) |
|---|---|---|
| 200 | 1.5 | Within 1 minute |
| 500 | 2.5 | Within 1 minute |
| 800 | 3.1 | Within 1 minute |

### Example 13

A spray-dried powder obtained in Example 4, erythritol, AC-di-sol and magnesium stearate were compounded so as to be 60 % by weight, 34 % by weight, 5 % by weight and 1 % by weight, respectively and mixed together. Thereafter, tablets each weighing 500 mg (tablet diameter:11.3 mm) were obtained. The relationship between molding pressure and hardness, and intraoral disintegration property (without chewing) are shown in the following table.

**Table 16**

| Molding Pressure (kg) | Hardness (kg) | Intraoral Disintegration Time (min) |
|---|---|---|
| 500 | 1.5 | Within 1 minute |
| 750 | 2.5 | Within 1 minute |
| 1000 | 3.1 | Within 1 minute |

From the results shown in the above, the intraoral disintegration time is within 1 minute, thus showing sufficient disintegration property [instantaneously (quickly) dissolution property] even when tabetting was done under molding pressure required generally to impart a necessary hardness to a tablet.

### Example 14

To 500 ml of water under stirring was added 120 g of erythritol and then 30 g of aldioxa, 60 g of magnesium alumino silicate "Neusilin A" (a trade name, a product of Fuji Chemical Industry Co., Ltd.) and 36 g of scaly calcium hydrogen phosphate "Fujicalin" were added thereto, followed by stirring and subjected to dispersion treatment with Mycolloider. The resultant suspension was spray-dried at an inlet temperature of 250 °C and an outlet temperature of 81 °C to obtain 183 g of powders (77.4 % recovery).

### Example 15

To 500 ml of water under stirring was added 120 g of xylitol and then 30 g of aldioxa, 60 g of magnesium alumino silicate "Neusilin A" and 36 g of scaly calcium hydrogen phosphate "Fujicalin" were added thereto, followed by stirring and subjected to dispersion treatment with Mycolloider. The resultant suspension was spray-dried at an inlet temperature of 250 °C and an outlet temperature of 91 °C to obtain 129 g of powders (54.2 % recovery).

### Example 16

To 500 ml of water under stirring was added 120 g of sorbitol and then 30 g of aldioxa, 60 g of magnesium alumino silicate "Neusilin A" and 36 g of scaly calcium hydrogen phosphate "Fujicalin" were added thereto, followed by stirring and subjected to dispersion treatment with Mycolloider. The resultant suspension was spray-dried at an inlet temperature of 250 °C and an outlet temperature of 90 °C to obtain 129 g of powders (40.5 % recovery).

### Comparative Example 3

To 420 ml of water under stirring was added 120 g of erythritol and then 30 g of aldioxa, 60 g of magnesium alumina silicate "Neusilin A" were added thereto, followed by stirring and subjected to dispersion treatment with Mycolloider. The resultant suspension was spray-dried at an inlet temperature of 250 °C and an outlet temperature of 90 °C to obtain 136 g of powders (66 % recovery).

### Comparative Example 4

To 420 ml of water under stirring was added 120 g of xylitol and then 30 g of aldioxa, 60 g of magnesium alumino silicate "Neusilin A" were added thereto, followed by stirring and subjected to dispersion treatment with Mycolloider. The resultant suspension was spray-dried at an inlet temperature of 250 °C and an outlet temperature of 72 ∼ 82 °C to obtain 16.1 g of powders (7.9 % recovery).

### Comparative Example 5

To 420 ml of water under stirring was added 120 g of sorbitol and then 30 g of aldioxa, 60 g of magnesium alumino silicate "Neusilin A" were added thereto, followed by stirring and subjected to dispersion treatment with Mycolloider. The resultant suspension was spray-dried at an inlet temperature of 250 °C and an outlet temperature of 75 ∼ 80 °C to obtain 21.2 g of powders (11.5 % recovery).

The yields of the saccharide-containing compositions obtained in Examples 14 ∼ 16 were as shown in table 17.

For comparison, the results prepared without adding calcium hydrogen phosphate are shown in Comparative Examples 3 ∼ 5.

**Table 17**

| | Saccharide | Calcium Hydrogen Phosphate | Yield (%) |
|---|---|---|---|
| Example 14 | Erythritol | "Fujicalin" | 77.4 |
| Example 15 | Xylitol | "Fujicalin" | 54.2 |
| Example 16 | Sorbitol | "Fujicalin" | 40.5 |
| Comparative Example 3 | Erythritol | - | 66.0 |
| Comparative Example 4 | Xylitol | - | 7. 9 |
| Comparative Example 5 | Sorbitol | - | 11.5 |

It can be seen that the yield may be significantly improved by spray-drying in the prsence of calcium hydrogen phosphate.

### INDUSTRIAL APPLICABILITY

According to the present invention, there can be provided a powdery composition containing calcium hydrogen phosphate and saccharide which has characteristic properties such as (1) improvement in the moldability of a tablet, (2) improvement in fluidity (specifaclly, direct compression molded tablet has a good homogeneity in the ingredient content), (3) improvement in taking feeling [refreshing sensation (cooling sensation)], (4) improvement in disintegration property, (5) disintegration property required for intraoral instantaneous (quick) dissoluble tablet and the like.

The composition of the present invention may be obtained by a simple and convenient process wherein a suspension comprising water medium, calcium hydrogen phosphate and a saccharide is spray-dried. Also, as the composition having the desired particle size may be prepared by altering the spray-drying condition properly, it is able to be flowed into the dies of high speed tabletting machine without causing bridging which occurs often with fine powders. Furthermore, it may be obtained as a homogenious particle in a large amount in a short time. The composition of the present invention has features that it is non-hygroscopic and stable under the normal condition and that it has little friction coefficient and that it is soft and has a good stability of particle.

The composition of the present invention is useful as an excipient, etc.

## Claims

1. A composition containing a saccharide and calcium hydrogen phosphate which is in a powdery form and which is prepared by spray-drying a suspension comprising water medium, calcium hydrogen phosphate and a saccharide

2. The composition as claimed in claim 1 wherein calcium hydrogen phosphate present in the suspension to be spray-dried is a scaly calcium hydrogen phosphate.

3. The composition as claimed in claim 1 wherein the saccharide is erythritol.

4. A process for preparing the composition as claimed in claim 1 which is characterized by spray-drying a suspension comprising water medium, calcium hydrogen phosphate and a saccharide.

5. An excipient comprising as the main ingredient the composition as claimed in any of claims 1 ∼ 3.

6. A compression moldability-improving agent of a phamaceutical preparation which comprises as the main ingredient the composition as claimed in any of claims 1 ∼ 3.

7. A disintegration aid comprising as the main ingredient the composition as claimed in any of claims 1 ∼ 3.

8. A taking feeling-improving agent comprising as the main ingredient the composition as claimed in any of claims 1 ∼ 3.

9. An intraoral instantaneous (quick) dissoluble agent comprising as the main ingredient any one of the composition as claimed in any of claims 1 ∼ 3.

10. The intraoral instantaneous (quick) dissoluble agent as claimed in claim 9 which is characterized by containing further a saccharide.
